# FASCICULE DE BREVET EUROPEEN

(11) **EP 1 587 560 B1**
(45) Date de publication et mention de la délivrance du brevet: **20.07.2011**
(21) Numéro de dépôt: 04702345.2
(22) Date de dépôt: 15.01.2004
(51) Int. Cl.: A61M 5/145

(54) **DISPOSITIF DE SUPPORT DE SERINGUE ANGIOGRAPHIQUE ET SES COMBINAISONS AVEC UNE SERINGUE ANGIOGRAPHIQUE ET AVEC UN INJECTEUR ANGIOGRAPHIQUE.**
VORRICHTUNG ZUM HALTEN EINER ANGIOGRAPHIE-SPRITZE UND IHRE KOMBINATION MIT EINER ANGIOGRAPHIE-SPRITZE UND MIT EINEM ANGIOGRAPHIE-INJEKTOR
ANGIOGRAPHIC SYRINGE SUPPORT DEVICE AND THE COMBINED USE THEREOF WITH AN ANGIOGRAPHIC SYRINGE AND AN ANGIOGRAPHIC INJECTOR

(30) Priorité: 28.01.2003 FR 0300927
(43) Date de publication de la demande: 26.10.2005
(73) Titulaire: SEDAT, 69540 Irigny (Rhône) (FR)
(72) Inventeur: BONACCI, Fabrice, F-69800 St Priest (FR)
(74) Mandataire: Jacobson, Claude
(86) Numéro de dépôt international: PCT/FR2004/000075
(87) Numéro de publication internationale: WO 2004/078237

(56) Documents cités:
- EP-A- 0 893 133
- WO-A-95/13841
- WO-A-02/056945
- US-A- 5 535 746

## Description

La présente invention est relative à un dispositif de support de seringue à chargement frontal pour un injecteur angiographique, ce dispositif étant conforme au préambule de la revendication 1.

L'invention concerne essentiellement le domaine de l'injection de produits de contraste pour l'établissement de diagnostics par imagerie médicale (scanners, imagerie par résonance magnétique ou IRM, et analogues).

Les seringues à chargement frontal sont munies, généralement à l'arrière, d'au moins un relief en saillie sur leur corps cylindriques afin de permettre leur fixation amovible sur l'injecteur ou sur un dispositif de support fixé sur la face avant de celui-ci. Le relief peut être un flasque (voir par exemple le WO-A-02/056 947) ou une paire de pattes diamétralement opposées (voir par exemple le WO-A-97/06 635).

Toutefois, les agencements ci-dessus ne sont pas entièrement satisfaisants, soit parce qu'ils n'assurent pas directement un positionnement angulaire de la seringue autour de son axe, soit parce que le mouvement de la seringue sur l'injecteur est relativement complexe, notamment du type baïonnette.

Le WO-A-95/13 841, grâce à un dispositif de support conforme au préambule de la revendication 1, fournit des moyens de fixation de la seringue en un seul geste qui assurent simultanément un positionnement annulaire de la seringue autour de son axe. Cependant, avec cet agencement connu, la déconnexion et le retrait de la seringue nécessitent un mouvement relativement complexe et peu précis.

L'invention a pour but de faciliter la déconnexion et le retrait de la seringue.

A cet effet, l'invention a pour objet un dispositif de support tel qu'indiqué plus haut, caractérisé par la partie caractérisante de la revendication 1.

Le dispositif de support selon l'invention peut comporter une ou plusieurs des caractéristiques des revendications 2 et 3.

L'invention a également pour objet un dispositif d'injection angiographique suivant la revendication 4.

L'invention a encore pour objet un système d'injection angiographique suivant la revendication 5.

D'autres caractéristiques de ce système d'injection angiographique sont décrites dans les revendications 6 à 8.

Des exemples de réalisation de l'invention vont maintenant être décrits on regard des dessins annexés, sur lesquels :
- la Figure 1 est une vue partielle schématique de face d'un système d'injection angiographique voisin de l'invention, la seringue n'étant pas représentée ;
- la Figure 2 est une vue partielle en coupe longitudinale du système de la Figure 1, avant raccordement de la seringue, la coupe étant prise suivant la ligne II-II de la Figue 4 ;
- la Figure 3 est une vue en perspective arrière du même système ;
- la Figure 4 est une vue de dessus du même système, prise suivant la flèche IV de la Figure 2 ;
- les Figures 5 et 6 sont des vues en perspective de l'ensemble seringue-dispositif de support-poussoir, respectivement de l'arrière et de l'avant, avant connexion de la seringue au poussoir ;
- les Figures 7 et 8 sont des vues analogues respectivement aux Figures 5 et 6, après raccordement de la seringue au poussoir ;
- les Figures 9, 10 et 12 sont des vues d'un système d'injection angiographique conforme à l'invention, respectivement de l'avant, en coupe longitudinale suivant la ligne x-x de la Figure 12, et de dessus, après fixation de la seringue sur l'injecteur;
- la Figure 11 est une vue partielle en perspective de l'arrière du système des Figures 9, 10 et 12, la face avant de l'injecteur étant omise; et
- les Figures 13 à 16 sont des vues correspondant respectivement aux Figures 9 à 12, après déconnexion de la seringue et du poussoir.

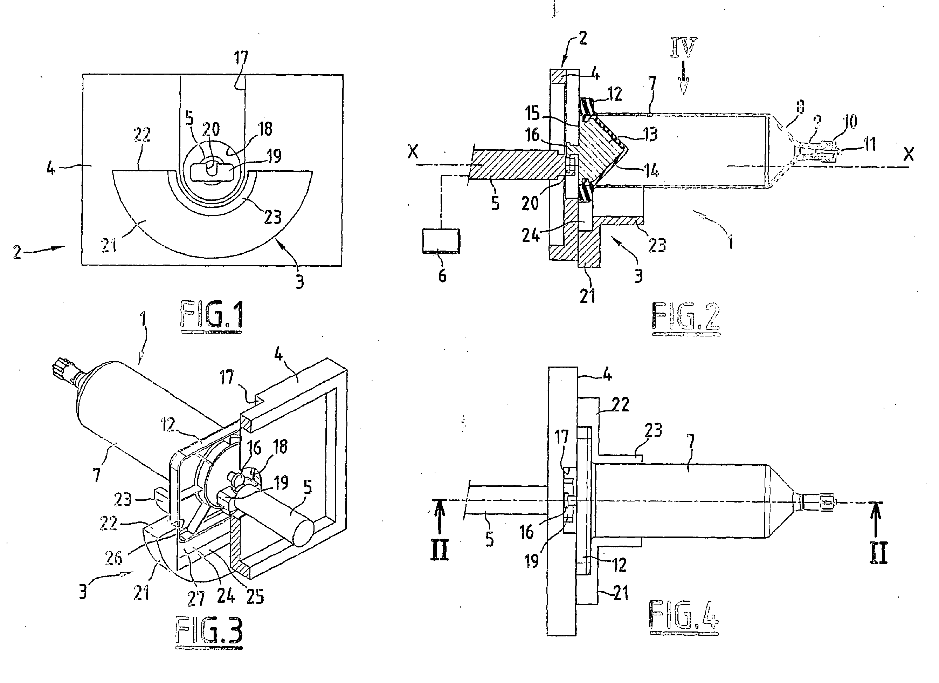

Le système d'injection angiographique représenté aux Figures 1 à 8 est constitué essentiellement d'une seringue angiographique 1, d'un injecteur angiographique 2 et d'un dispositif 3 de support de la seringue, fixé sur la face avant 4 de l'injecteur. Ce dernier comprend un poussoir 5 mobile en translation suivant son axe X-X sous la commande de moyens de commande 6 illustrés très schématiquement. Seuls la face avant 4 et le poussoir 5 de l'injecteur ont été représentés.

La seringue 1 comprend un corps cylindrique 7 dont la partie avant 8 converge jusqu'à un conduit de sortie 9 équipé d'un raccord 10 pour un tube souple 11. L'extrémité arrière du corps 7 est pourvu d'une collerette extérieure radiale 12, de forme extérieure sensiblement rectangulaire.

Dans le corps 7 est disposé un piston ou porte-joint 13. La face avant de ce piston est recouverte d'un joint élastomère 14 et a une forme conique conjuguée de la partie avant 8 du corps de la seringue. Le joint 14 se prolonge vers l'arrière de manière à coopérer avec frottement avec la paroi intérieure du corps. La face arrière 15 du piston est plane et munie en son centre d'un pion 16 en saillie vers l'arrière, en forme de champignon à section circulaire.

La face avant 4 de l'injecteur est plane. Elle comporte un évidement en U 17 à axe vertical muni à sa base d'un orifice circulaire 18 d'axe X-X, adapté pour être traversé librement par le poussoir 5.

Le poussoir 5, dont la section courante est circulaire, comporte une tête avant 19 de forme générale rectangulaire à grands côtés horizontaux. Dans le côté supérieur de cette tête est ménagé un logement à gradin 20 conjugué de la moitié inférieure du pion 16.

Le dispositif de support 3 est constitué d'un demi-disque 21 délimité par une surface supérieure horizontale 22 et prolongé vers l'avant par un berceau semi-cylindrique 23, ouvert vers le haut. Le demi-disque et le berceau sont réalisés en une seule pièce. Le demi-disque 21 comporte, sur environ la moitié arrière de son longueur, un évidement 24 ouvert vers l'arrière, dont la section transversale est conjuguée de celle de la moitié inférieure du flasque 12 de la seringue lorsque les grands côtés de celui-ci sont horizontaux. L'évidement 24 est ainsi délimité par une face inférieure horizontale 25, par deux parois verticales 26 en regard, et par une face avant 27 verticale. Le berceau 23 débouche directement dans la face avant 27.

Le dispositif de support 3 est fixé sur la face avant 4 de l'injecteur de manière que l'axe du berceau 23 soit confondu avec l'axe x-x. Le pourtour inférieur de l'évidement 17 définit alors la paroi arrière de l'évidement 24, lequel a la même longueur axiale que le flasque 12 de la seringue.

La fixation de la seringue sur l'injecteur s'effectue comme suit.

Le poussoir 5 étant dans sa position rétractée des Figures 2 et 3, en léger retrait par rapport à la face avant de l'injecteur, le grand côté inférieur du flasque 12 de la seringue est posé sur la face supérieure du berceau 23, et la seringue est poussée vers l'arrière.

Lorsque le flasque bute contre la face avant 4 de l'injecteur, il se trouve juste au-dessus de l'évidement 24, et le pion 16 se trouve juste au-dessus du logement 20 du poussoir :

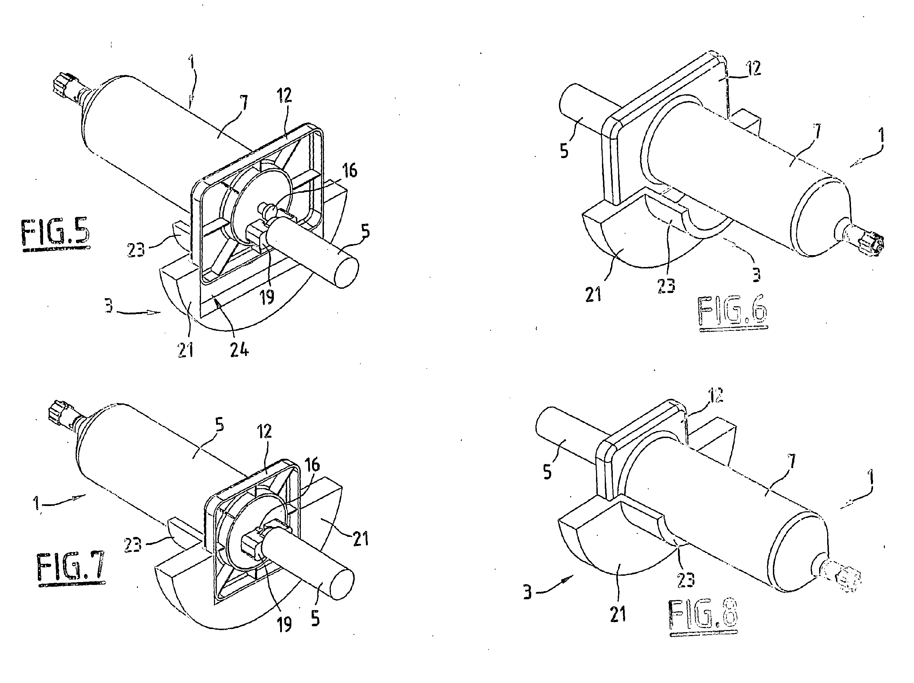

Un simple déplacement vers le bas de la seringue amène alors simultanément la partie inférieure du flasque 12 dans l'évidement 24, celle du corps de seringue 7 dans le berceau 23, et celle du pion 16 dans le logement 20 (Figures 7 et 8).

Ainsi, la seringue est bloquée en translation par les faces 4 et 27, son corps est soutenu par le berceau 23, et le pion 16 est connecté, pour les deux sens d'entraînement, à la tête de poussoir 19. Aucune pièce mobile de fixation de la seringue n'est nécessaire.

L'actionnement opérationnel du poussoir peut donc commencer immédiatement.

Pour déconnecter la seringue de l'injecteur, il suffit, le poussoir étant rétracté, de soulever la seringue, puis, lorsque le flasque est entièrement sorti de l'évidement 24, d'extraire la seringue vers l'avant.

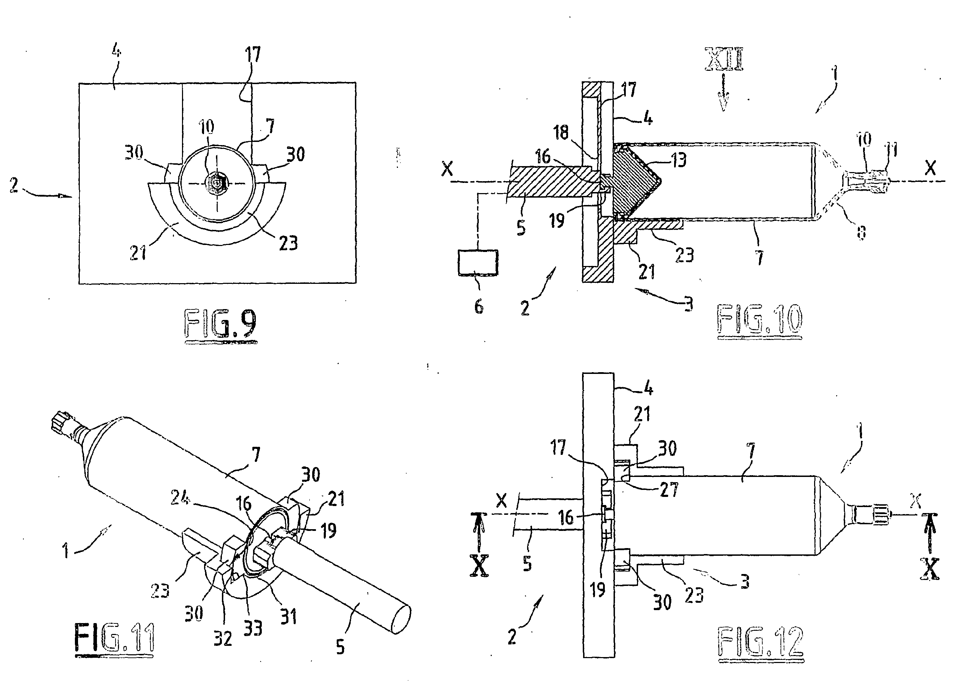

Le système d'injection angiographique des Figures 9 à 12 ne diffère de celui des Figures 1 à 8 que par les points suivants.

D'une part, le flasque de la seringue est remplacé par deux pattes radiales 30 diamétralement opposées.

D'autre part, l'évidement 24 du dispositif de support est constitué par une partie centrale 31 à section en arc de cercle, qui prolonge la surface intérieure du berceau 23, et par deux encoches horizontales 32, diamétralement opposées, qui débouchent dans la partie centrale 31 et qui sont sensiblement conjuguées de la moitié inférieure des pattes 30. Chaque surface 33 de raccordement d'une encoche 32 à la partie centrale 31 est une surface courbe convexe (Figure 11).

La fixation de la seringue sur l'injecteur s'effectue comme décrit plus haut en regard des Figures 1 à 8, à ceci près que ce sont les surfaces inférieures des deux pattes 30 qui glissent sur les surface supérieures du berceau 23. De nouveau, la connexion du pion 16 et de la tête de poussoir 19 est obtenue simultanément.

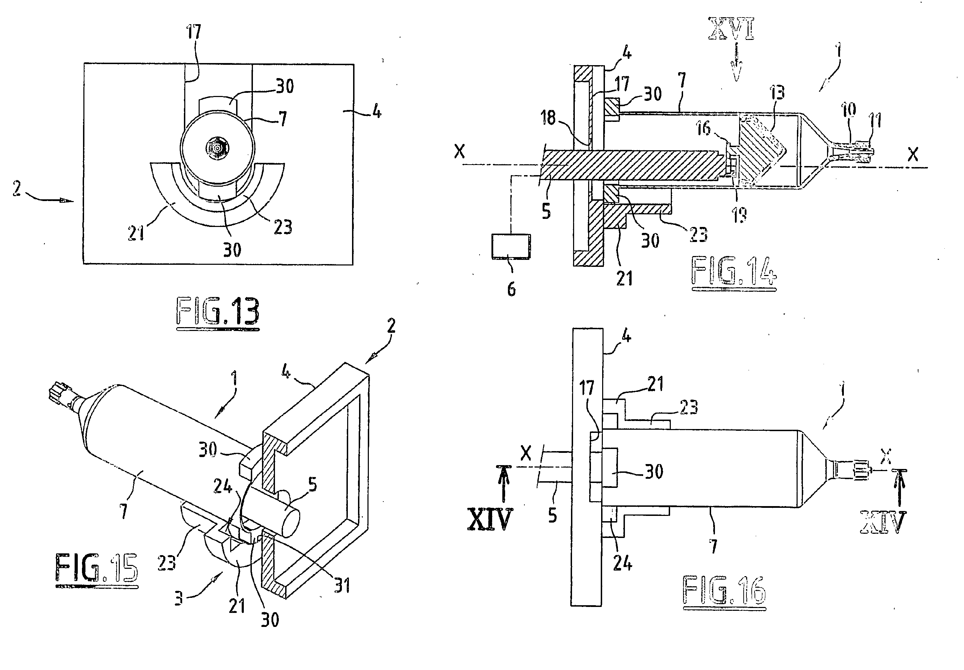

Pour déconnecter la seringue, on saisit son corps 7 et on le tourne de 90°. L'une des pattes 30 coopère alors avec le fond de l'encoche 31 puis avec la surface convexe 33 associée, laquelle forme une rampe de came, ce qui provoque le soulèvement de la seringue et, par suite, la déconnexion du piston et du poussoir..Les deux pattes sont ainsi amenées dans un plan général vertical (Figures 13 à 15), et la seringue peut être tirée par simple traction vers l'avant.

Cette variante permet de faciliter la déconnexion piston-poussoir. De plus, la configuration des deux pattes et des surfaces avant 27 de butée correspondantes du dispositif de support permet de retirer la seringue vers l'avant même si le poussoir est engagé dans le corps de la seringue, ceci sans avoir à effectuer préalablement une course de retrait de ce poussoir.

## Revendications

1. Dispositif de support de seringue à chargement frontal pour un injecteur angiographique, ce dispositif étant adapté pour être fixé sur la face avant (4) de l'injecteur (2) et pour positionner une seringue angiographique (1) dont le corps (7) présente un relief extérieur (30), la section transversale du corps à l'emplacement de ce relief étant non circulaire, le dispositif comportant un évidement (24) de réception dudit relief, cet évidement étant ouvert dans une direction de réception, notamment vers le haut, et présentant d'une part une section transversale non circulaire conjuguée d'une partie de la section transversale du corps de seringue à l'emplacement dudit relief, et d'autre part une face avant (27) de butée pour ledit relief, le dispositif se prolongeant vers l'avant par un berceau (23) de soutien du corps de seringue, **caractérisé en ce que** l'évidement (24) comporte une partie centrale (31) à section transversale en arc de cercle, qui se prolonge par deux encoches diamétralement opposées (32), et **en ce que** ladite partie centrale (31) prolonge la surface intérieure du berceau (23).

2. Dispositif de support suivant la revendication 1, **caractérisé en ce que** chaque encoche (32) est reliée à la partie centrale (31) par une surface courbe convexe (33) formant came.

3. Dispositif de support suivant la revendication 1 ou 2, **caractérisé en ce que** l'évidement (24) est ouvert vers l'arrière.

4. Dispositif d'injection angiographique, **caractérisé en ce qu'**il comprend :
- une seringue angiographique (1) dont le corps (7) est muni d'un relief (30) en saillie extérieure, la section transversale du corps à l'emplacement de ce relief étant non circulaire, ledit relier (30) étant constitué par deux pattes diamétralement opposées dont chacune est adaptée pour être reçue dans l'une des encoches (32) de manière à être positionnée par celle-ci ; et
- un dispositif (3) de support de seringue suivant l'une quelconque des revendications 1 à 3.

5. Système d'injection angiographique, du type comprenant un injecteur angiographique (2) comportant un poussoir (5) mobile axialement, au moins une seringue angiographique (1) comportant un piston (13) muni de moyens (16) de raccordement amovible à la tête avant (19) du poussoir, et des moyens de fixation amovible de la seringue, sur la face avant (4) de l'injecteur, **caractérisé en ce qu'**il comprend au moins un dispositif d'injection angiographique suivant la revendication 4, le dispositif (3) de support de seringue étant fixé sur la face avant (4) de l'injecteur.

6. Système d'injection suivant la revendication 5, **caractérisé en ce que** l'agencement est tel que, à partir de la position de fixation de la seringue, une rotation de 90° de celle-ci provoque le soulèvement de la seringue par coopération de l'une des pattes (30) avec le fond de l'encoche (32) associée et la déconnexion du piston (13) et du poussoir (5), la seringue pouvant alors être retirée verts l'avant même si le poussoir est engagé dans le corps de la seringue.

7. Système d'injection angiographique suivant la revendication 5, **caractérisé en ce que** le dispositif de support (3) est conforme à la revendication 3, et **en ce que** la face avant (4) de l'injecteur (2) forme la face arrière de l'évidement (24).

8. Système d'injection angiographique suivant l'une quelconque des revendications 5 à 7, **caractérisé en ce que** la tête (19) du poussoir (5) et le piston (13) comportent l'un une pion (16) à contre-dépouille et l'autre une fente (20) ouverte dans ladite direction de réception ou dans la direction opposée, de sorte que, en position rétractée du poussoir, la mise en place du relief (30) de la seringue (1) dans l'évidement (24) par un déplacement dans la direction opposée à ladite direction de réception provoque l'insertion du pion (16) dans la fente (20).

## Claims

1. Support device for a front-loaded syringe for an angiographic injector, which device is designed to be attached to the front face (4) of the injector (2) and for positioning an angiographic syringe (1), the body (7) of which has an external relief (30), the cross-section of the body in the region of this relief being non-circular, the device having a recess (24) for receiving said relief, this recess being open in a receiving direction, in particular at the top, and comprising, on the one hand, a non-circular cross-section complementing a part of the cross-section of the syringe body in the region of said relief, and on the other hand, a front abutment face (27) for said relief, the device being extended at the front by a cradle (23) for supporting the syringe body, **characterised in that** the recess (24) comprises a central part (31) with a cross-section in the shape of a circular arc which is extended by two diametrically opposite notches (32), and **in that** said central part (31) extends the internal surface of the cradle (23).

2. Support device as claimed in claim 1, **characterised in that** each notch (32) is connected to the central part (31) by a convex, curved surface (33) forming a cam.

3. Support device as claimed in claim 1 or 2, **characterised in that** the recess (24) is open at the rear.

4. Angiographic injection device, **characterised in that** it comprises :
- an angiographic syringe (1), the body (7) of which is provided with an eternally protruding relief (30), the cross-section of the body in the region of this relief being non-circular, said relief (30) being made up by two diametrically opposite lugs, each of which is designed to be received in one of the notches (32) so as to be positioned by the latter; and
- a syringe support device (3) as claimed, in any one of claims 1 to 3.

5. Angiographic injection system of the type comprising an angiographic injector (2) comprising an axially displaceable push rod (5), at least one angiographic syringe (1) comprising a plunger (13) provided with detachable means (16) for connecting to the front head (19) of the push rod and detachable means for attaching the syringe to the front face (4) of the injector, **characterised in that** it comprises at least an angiographic injection device as claimed in claim 4, the syringe support device (3) being attached to the front face (4) of the injector.

6. Infection system as claimed in claim 5, **characterised in that** the arrangement is such that from the fixed position of the syringe, a rotation of the latter by 90° causes the syringe to be lifted due to the co-operation of one of the lugs (30) with the base of the co-operating notch (32) and the plunger (13) and push rod (5) to be disconnected, it then being possible for the syringe to be retracted towards the front, even if the push rod is engaged in the body of the syringe.

7. Angiographic injection system as claimed in claim 5, **characterised in that** the support device (3) is as claimed in claim 3 and **in that** the front face (4) of the injector (2) constitutes the rear face of the recess (24).

8. Angiographic injection system as claimed in any one of claims 5 to 7, **characterised in that** the head (19) of the push rod (5) and the plunger (13) have an undercut pin (16) in the case of one of them and a slot (20) open in said receiving direction or in the opposite direction in the case of the other one, so that when the push rod is in the retracted position, placing the relief (30) of the syringe (1) in the recess (24) by a displacement in the direction opposite said receiving direction causes the pin (16) to be inserted in the slot (20).

## Patentansprüche

1. Abstützvorrichtung für eine Frontladungs-Spritze für einen Angiographie-Injektor, wobei die Vorrichtung angepasst ist, um an der Vorderseite (4) des Injektors (2) befestigt zu werden und um eine Angiographiespritze (1) zu positionieren, deren Körper (7) eine äußere Erhöhung (30) aufweist, wobei der Querschnitt des Körpers an der Stelle der Erhöhung nicht kreisförmig ist, wobei die "vorrichtung eine Aussparung (24) zum Aufnehmen der Erhöhung aufweist, wobei die Aussparung in einer Aufnahmerichtung insbesondere nach oben offen ist und auf der einen Seite einen nicht kreisförmigen, an der Stelle der Erhöhung mit einem Teil des Querschnitts des Spritzenkörpers verbundenen Teil, und auf der anderen Seite eine Anschlags-Vorderseite (27) für die Erhöhung aufweist, wobei sich die Vorrichtung über ein Gestell (23) zum Halten des Spritzenkörpers nach vorne verlängert, **dadurch gekennzeichnet, dass** die Aussparung (24) einen zentralen Teil (31) mit kreisbogenförmigem Querschnitt auf weist, der sich über zwei diametral entgegengesetzte Nuten (32) verlängert, und dass der zentrale Teil (31) die Innenfläche des Gestells (23) verlängert.

2. Abstützvorrichtung gemäß Anspruch 1, **dadurch gekennzeichnet, dass** jede Nut (32) über eine konvex gekrümmte Fläche (33), die einen Nocken ausbildet, mit dem zentralen Teil (31) verbunden ist.

3. Abstützvorrichtung gemäß Anspruch 1 oder 2, **dadurch gekennzeichnet, dass** die Aussparung (24) nach hinten offen ist.

4. Angiographie-Injektionsvorrichtung, **dadurch gekennzeichnet** das sie aufweist:
- eine Angiographiespritze (1), deren Körper (7) mit einer nach außen hervorstehenden Erhöhung (30) versehen ist, wobei der Querschnitt des Körpers an der Stelle der Erhöhung nicht kreisförmig ist, wobei die Erhöhung (30) von zwei diametral entgegengesetzten Klauen gebildet wird, von denen jede angepasst ist, um in einer der Nuten (32) aufgenommen zu werden, um mittels dieser positioniert zu werden, und
- eine Spritzen-Abstützvorrichtung (3) gemäß einem der Ansprüche 1 bis 3.

5. Angiographie-Injektionssystem des Typs, der aufweist:
einen Angiographie-Injektor (2), aufweisend einen axial bewegbaren Drücker (5), mindestens eine Angiographiespritze (1), aufweisend einen Kolben (13), der mit Mitteln (16) zum lösbaren Verbinden mit dem vorderen Kopfstück (19) des Drückers versehen ist, und Mittel zum lösbaren Befestigen der Spritze an der Vorderseite (4) des Injektors, **dadurch gekennzeichnet, dass** es mindestens eine Angiographie-Injektionsvorrichtung gemäß Anspruch 4 aufweist, wobei die Spritzen-Abstützvorrichtung (3) an der Vorderseite (4) des Injektors befestigt ist.

6. Injektionssystem gemäß Anspruch 5, **dadurch gekennzeichnet, dass** die Anordnung derart ist, dass ausgehend von der Befestigungsposition der Spritze eine Drehung von 90° derselben das Anheben der Spritze durch Zusammenwirken einer der Klauen (30) mit dem Boden der zugehörigen Nut (32) und das Lösen des Kolbens (13) und des Drückers (5) bewirkt, wobei die Spritze dann nach vorne herausgenommen werden kann, selbst wenn der Drücker mit dem Körper der Spritze in Eingriff ist.

7. Angiographie-Injektionssystem gemäß Anspruch 5, **dadurch gekennzeichnet, dass** die Abstützvorrichtung (3) dem Anspruch 3 entspricht, und dass die Vorderseite (4) des Injektors die Rückseite der Aussparung (24) bildet.

8. Angioraphie-Injektionssystem gemäß einem der Ansprüche 5 bis 7, **dadurch gekennzeichnet, dass** das Kopfstück (19) des Drückers (5) und der Kolben (13) einen hinterschnittenen Stift (16) beziehungsweise einen in der Aufnahmerichtung oder in der entgegengesetzten Richtung offenen Schlitz (20) aufweisen, so dass in der Zurückzieh-Position des Drückers das Platzieren der Erhöhung (30) der Spritze (1) in der Aussparung (24) durch ein Bewegen in der zu der Aufnahmerichtung entgegengesetzten Richtung das Einsetzen des Stiftes (16) in den Schlitz (20) bewirkt.
